# EUROPEAN PATENT APPLICATION

(11) **EP 1 642 555 A1**
(43) Date of publication of application: **05.04.2006**
(21) Application number: 05021325.5
(22) Date of filing: 29.09.2005
(51) Int. Cl.: A61F 9/013

(54) **Corneal surgical apparatus and blade unit attached to corneal surgical apparatus**

(30) Priority: 30.09.2004 JP 2004286051; 08.08.2005 JP 2005229697
(71) Applicant: Nidek Co., Ltd., Gamagori-shi, Aichi (JP)
(72) Inventor: Sugimura, Masahiro, Kota-cho, Nukata-gun, Aichi (JP)
(74) Representative: Hager, Thomas Johannes

(57) **Abstract**

A corneal surgical apparatus for forming a flap in a cornea, includes: a blade unit including a blade, and an identification information communicating portion that transmits identification information; a head portion that holds the blade unit detachably; an oscillation mechanism portion that oscillates the blade unit, which is held by the head portion, in a direction of a width of the blade; a receiving portion that receives the identification information transmitted from the identification information communicating portion; a storage portion that stores the identification information; anda control portion that collates the identification information received by the receiving portion with the identification information stored in the storage portion, and controls the driving of the oscillation mechanism portion based on a result of collation.

## Description

### BACKGROUND OF THE INVENTION

The present invention relates to a corneal surgical apparatus for forming a flap in a comea at a keratorefractive surgery, and to a blade unit, attached to the corneal surgical apparatus, for use in the keratorefractive surgery.

The keratorefractive surgery is known in which a flap is first formed in a cornea and subsequently, the formed flap is opened and then, the comea is irradiated with an excimer laser beam to thereby cause ablation of a stroma, and thereafter, the opened flap is returned to an original position. Inthis surgery, operative procedures or techniques, such as LASIK (Laser in Situ Keratomileusis) and Epi-LASIK, are performed. According to LASIK, a flap is formed by incising a corneal portion having a thickness of 150µm ranging from an epithelium to the stroma. According to Epi-LASIK, a flap is formed by separating only the epithelium. Further, a corneal surgical apparatus called "Microkeratome" has been proposed as an apparatus configured to form a flap in a cornea.

A corneal surgical apparatus enabled to be compatible with different techniques, such as LASIK and Epi-LASIK, only by replacing blades respectively corresponding to the different techniques has been proposed. However, the differences between the blades respectively corresponding to, for example, LASIK and Epi-LASIK are minute and reside in the shape of a cutting edge. Thus, there is a possibility that the blade corresponding to the technique differing from the technique to be performed may erroneously be attached to the surgical apparatus.

### SUMMARY OF THE INVENTION

An object of the invention is to provide a corneal surgical apparatus, which can prevent erroneous attachment of a blade thereto, and to a blade unit attached to the corneal surgical apparatus for use in a corneal surgery.

In order to solve the afore the object, the invention is characterized by having the following arrangement.
(1) A corneal surgical apparatus for forming a flap in a cornea, comprising:
   a blade unit including a blade, and an identification information communicating portion that transmits identification information;
   a head portion that holds the blade unit detachably;
   an oscillation mechanism portion that oscillates the blade unit, which is held by the head portion, in a direction of a width of the blade;
   a receiving portion that receives the identification information transmitted from the identification information communicating portion;
   a storage portion that stores the identification information; and
   a control portion that collates the identification information received by the receiving portion with the identification information stored in the storage portion, and controls the driving of the oscillation mechanism portion based on a result of collation.
(2) The corneal surgical apparatus according to (1), wherein the identification information communicating portion transmits intended-use information of the blade unit as the identification information, and the storage portion stores the intended-use information of the blade unit as the identification information.
(3) The corneal surgical apparatus according to (2) further comprising a selection switch for selecting a corneal surgical technique,
   wherein the control portion reads the intended-use information from the storage portion based on the selected technique and collates the received intended-use information with the read intended-use information.
(4)4 The corneal surgical apparatus according to (1), wherein the identification information communicating portion transmits manufacturing information of the blade unit as identification information, and the storage portion stores the manufacturing information of the blade unit as identification information.
(5) The corneal surgical apparatus according to (1) further comprising a notification portion,
   wherein the control portion controls the notification portion to notify a result of collation.
(6) The corneal surgical apparatus according to (1) further comprising a translation mechanism portion that moves the blade unit, which is held by the head portion, in a direction in which the flap is to be formed,
   wherein the control portion controls driving of the translation mechanism portion based on a result of collation.
(7) The corneal surgical apparatus according to (6), wherein
   the identification information communicating portion transmits driving condition information including at least one of an oscillation frequency and a translation speed of the blade unit,
   the receiving portion receives the driving condition information transmitted from the identification information communicating portion, and
   the control portion controls driving of at least one of the oscillation mechanism portion and the translation mechanism portion based on driving condition information received by the receiving portion.
(8) A blade unit attached to a corneal surgical apparatus for use in forming a flap in a cornea, comprising:
   a blade;
   a transmission member that is fixed to the blade and transmits to the blade impetus applied from the corneal surgical apparatus and causes the blade to oscillate in a direction of a width of the blade when the blade unit is attached to the corneal surgical apparatus; and
   an identification information communicating portion that is provided in the transmission member and transmits identification information.
(9) The blade unit according to (8), wherein the identification information communicating portion transmits at least one of intended-use information and manufacturing information of the blade unit.
(10) The blade unit according to (8), wherein the identification information communicating portion transmits driving-condition information including an oscillation frequency of the blade unit .

### BRIEF DESCRIPTION OF THE DRAWINGS

FIG. 1 is a schematic view illustrating a configuration of a corneal surgical apparatus according to an embodiment of the invention.
FIG. 2 is a schematic view illustrating a configuration of a blade unit, which is attached to the corneal surgical apparatus, for use in a corneal surgery.
FIG. 3 is an enlarged view of a part of the corneal surgical apparatus shown in FIG. 1.
FIG. 4A is an enlarged cross-sectional view taken along line A-A shown in FIG. 3. FIG. 4B is an enlarged cross-sectional view taken along line B-B shown in FIG. 3.

### DETAILED DESCRIPTION OF PREFERRED EMBODIMENTS

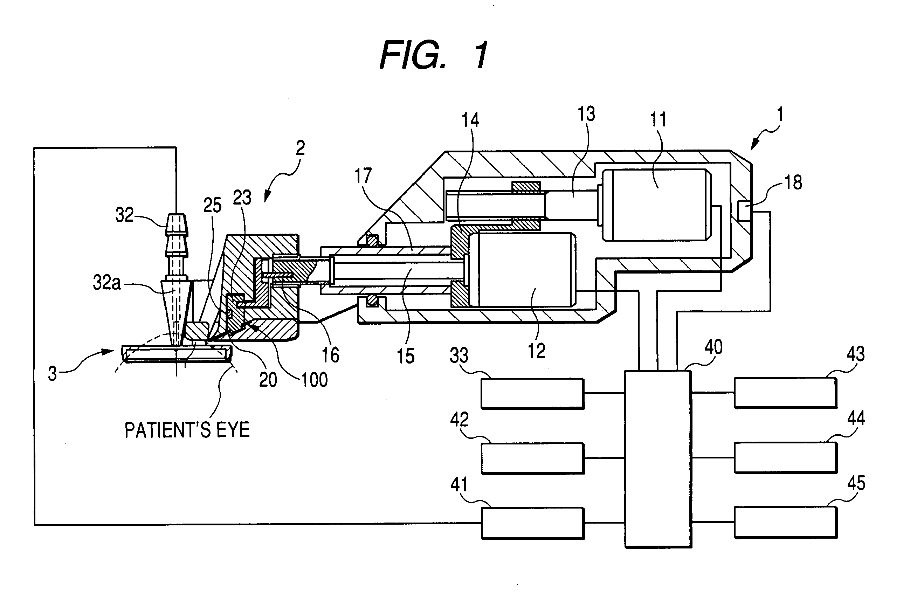

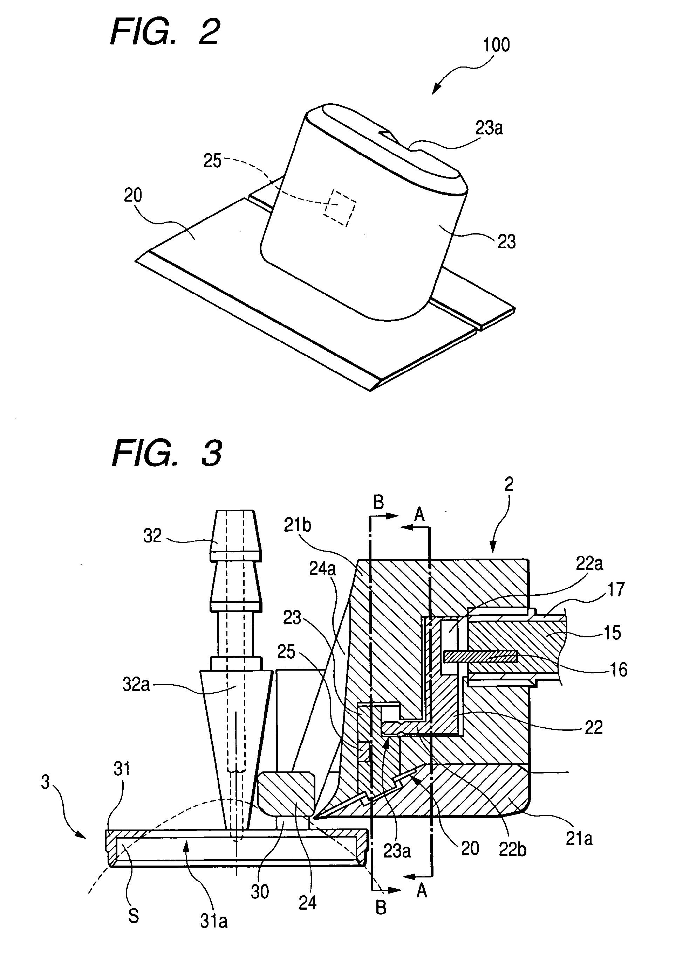

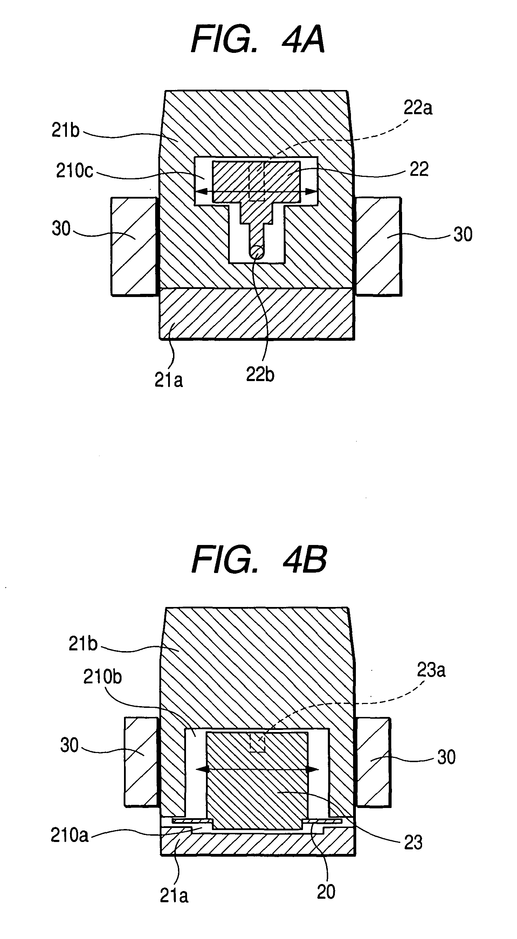

Hereinafter, an embodiment of the invention is described with reference to the accompanying drawings. FIG. 1 is a schematic view illustrating the configuration of a corneal surgical apparatus according to an embodiment of the invention. FIG. 2 is a schematic view illustrating the configuration of a blade unit, which is attached to this corneal surgical apparatus, for use in a corneal surgery. FIG. 3 is an enlarged view of a part of the corneal surgical apparatus shown in FIG. 1. FIG. 4A is an enlarged cross-sectional view taken along line A-A shown in FIG. 3. FIG. 4B is an enlarged cross-sectional view taken along line B-B shown in FIG. 3.

Reference numeral 1 designates a body portion (a hand piece portion) of the corneal surgical apparatus. A suction portion 3 for fixing the body portion 1 to a patient's eye, and a head portion 2 for holding a blade unit 100 (or a blade 20) for forming a flap in a cornea of the patient's eye, which is rectilinearly moved (translated) on the suction portion 3, are provided at the front side (the left side, as viewed in FIG. 1) of the body portion 1.

The blade unit 100 includes the blade 20, and a first oscillation transmission member 23 fixed to the blade 20. A metal blade made of stainless steel, steel, or the like, a mineral blade made of diamond, sapphire, or the like, a resin blade, a ceramic blade or the like are utilized as the blade 20. Further, LASIK blade unit 100, which includes LASIK blade 20 having a sharp edge, and Epi-LASIK blade unit 100, which includes Epi-LASIK blade 20 whose edge is not sharp as that of the LASIK blade 20, are available as the blade unit 100.

A longitudinal (vertical) groove 23a to be engaged with a protrusion portion 22b of a second oscillation transmission member 22, which will be described later, is formed in the back surface of the first transmission member 23. Further, an identification information communicating portion 25 including an antenna coil and an IC chip is disposed (embedded in this embodiment) in the fist transmission member 23. Incidentally, the communicating portion 25 may be configured by incorporating an antenna in the IC chip. Identification information concerning the blade unit 100 (the blade 20), which includes information on the intended use, such as the corresponding technique, of the blade unit 100 (the blade 20) and the manufacturing information, such as representing the serial number, of the blade unit 100 (the blade 20) is stored in the IC chip of this communicating portion 25. Further, information can be read from and written to the IC chip used in this embodiment.

Amotor 11 for rectilinearly moving (translating) the head portion 2 in the direction of incision of the cornea (or the direction of separation of an epithelium), and a motor 12 for oscillating the blade unit 100 (the blade 20) in the direction of width of the blade, are incorporated in the body portion 1. A connecting member 14 is screwed onto a lead screw 13 fixed to the rotation shaft of the motor 11. The motor 12 and a connecting member 17 are fixed to the connecting member 14. The motor 12 and the connecting member 17 are rectilinearly moved (translated) forwardly and rearwardly by the positive rotation and the negative rotation of the motor 11 through the screw 13 and the connecting member 14. Consequently, a rotating shaft 15 fixed to the rotation shaft of the motor 12 is rotatably held by the connecting member 17. An eccentric pin 16 is implanted at a place located eccentrically from the center of rotation of the shaft 15 in an end portion thereof.

The head portion 2 includes a blade holder 21a and a holder block 21b for holding the blade unit 100 (the blade 20) oscillatably, the second transmission member 22 for converting the circumferential motions of the pin 16 caused by the rotation of the shaft 15 into oscillation and transmitting the oscillation to the blade unit 100 (the blade 20), and a cornea applanating member 24 for applanating the cornea substantially flat before the blade 20 acts thereon. Incidentally, a hole, into which the shaft 15 is inserted, is provided in the block 21b to which an end portion of the connecting member 17 is fixed.

A shallow concave portion 210a is formed in a part of the holder 21a, on which the blade 20 is put. The width of the concave portion 210a is set to be larger than the oscillation width (range) of the blade 20. The holder 21a is detachably attached to the block 21b by screws (not shown). The blade unit 100 can be detached by removing the holder 21a.

The second transmission member 22 is held in a receiving groove 210c that is formed in the block 21b. A longitudinal (vertical) groove 22a engaged with the pin 16 is formed in the second transmission member 22. When the shaft 15 is rotated by rotate-driving of the motor 12, impetus acting in a direction (hereunder referred to as a lateral direction) perpendicular to the rotation axis of the shaft 15 is applied to the second transmission member 22 by the circumferential motion of the pin 16 engaged with the longitudinal groove 22a. Consequently, the second transmission member 22 is oscillated in the lateral direction.

The first transmission member 23 is oscillatably held in a receiving groove 210b formed by the holder 21a and the block 21b. The protrusion portion 22b is formed on a lower part of the second transmission member 22 and is engaged with the longitudinal groove 23a formed in the first transmission member 23. When the second transmission member 22 is laterally oscillated by the rotation of the shaft 15 (the circumferential motion of the pin 16), lateral impetus is also applied to the first transmission member 23 by the oscillation of the protrusion portion 22b engaged with the longitudinal groove 23a. Consequently, the first transmission member 23 is laterally oscillated. Further, the blade 20 fixed to the first transmission member 23 is laterally oscillated.

The cornea applanating member 24 is provided at the front side (the left side, as viewed in FIG. 3) of the blade 20 by a mounting member 24a. The cornea applanating member 24 applanates the cornea substantially flat before the blade 20 acts thereon.

The suction portion 3 includes a suction ring 31 and a suction pipe 32. The substantially cylindrical suction ring 31 made to abut against a patient's eye is fixed to the body portion 1 by a fixing member 30. An opening portion 31a is formed in the ring 31. When the ring 31 is placed on the patient' s eye, the cornea projects from the opening portion 31. The bottom of the ring 31 and the opening end of the opening portion 31a are made to abut against the patient's eye thereby to form a suction space S. The pipe 32 is fixed to the ring 31 and is connected to the pump 41 through a tube (not shown) . The ring 31 is suction-fixed to the patient's eye by driving the pump 41 to suck air in the space S through a suction passage 32a in the pipe 32.

Further, a pressure detector 33 is connected to the ring 31 through a pipe and a tube, which are not shown, and detects the air pressure in the air in the space S. An arithmetic control portion 40 controls the driving of the pump 41, and the motors 11 and 12 according to the air pressure detected by the pressure detector 33.

A transmitter-receiver portion 18 receives the identification information stored in the IC chip of the communicating portion 25 by wireless communication. The identification information received by the transmitter-receiverportion 18 is sent to the arithmetic control portion 40 incorporated in a control box, which is separated from the body portion 1, through a connecting cable. Incidentally, although the transmitter-receiver portion 18 is disposed in the body portion 1 of this embodiment, the placement of the transmitter-receiver portion 18 is not limited thereto. It is advisable to place the transmitter-receiver portion 18 at a position at which the transmitter-receiver portion 18 can receive a signal from the communicating portion 25. As long as the transmitter-receiver portion 18 can communicate with the communicating portion 25, the transmitter-receiver portion 18 may be disposed in the control box. Furthermore, the transmitter-receiver portion 18 also serves to transmit various kinds of information to the communicating portion 25.

The motors 11 and 12, the transmitter-receiver portion 18, the pressure detector 33, the pump 41, a foot switch 42, a memory 43, a switch portion 44, and a display unit 45 are connected to the arithmetic control portion 40.

The memory 43 stores the identification information concerning the blade unit 100 (the blade 20), a drive program used to set different driving conditions (an oscillation frequency, a translation speed, and so forth) corresponding to the blade unit 100, and so on. The drive program sets the oscillation frequency and the translation speed of the Epi-LASIK blade unit 100 (the blade 20) to be lower than those of the LASIK blade unit 100 (the blade 20), respectively. Incidentally, both the oscillation frequency and the translation speed are changed according to the blade unit 100 (the blade 20) in this embodiment. However, only one of the oscillation frequency and the translation speed may be changed according to the blade unit 100 (the blade 20).

The switch portion 44 is provided with a selection switch used to select the technique such as the LASIK or the Epi-LASIK, a setting switch used to set the oscillation frequency and the translation speed of the blade unit 100 (the blade 20), a test switch used to check the blade unit 100 (the blade 20), and so on. The display unit 45 indicates the selected technique, a result of the detection by the pressure detector 33, information notified to an operator, and so forth.

An operation of the aforementioned corneal surgical apparatus is described below. First, an operator selects the technique by using the switching portion 44. When the technique is selected, the arithmetic control portion 40 reads from the memory 43 the identification information including the information on the intended use and the manufacturing information of the blade unit 100 (the blade 20) and the drive program corresponding to the selected technique.

Subsequently, the head portion 2 is removed from the body portion 1. The blade unit 100 corresponding to the selected technique is mounted in the head portion 2. Then, the head portion 2, in which the blade unit 100 is mounted, is attached to the body portion 1. Also, the test switch is turned on. While the test switch is on, when a trigger signal used to start collation is issued, the arithmetic control portion 40 causes the transmitter-receiver portion 18 to generate a magnetic field. The generation of the magnetic field results in the generation of electric current in the antenna coil of the communicating portion 25. Thus, the IC chip operates, so that the identification information of the blade unit 100 (the blade 20) is sent to the transmitter-receiver portion 18 through the antenna coil. Incidentally, although the transmission and the reception of the information are performed in this embodiment by causing the transmitter-receiver portion 18 to generate the magnetic field, the method of performing the transmission and the reception of the information is not limited thereto. The transmission and the reception of the information may be performed by using microwaves.

The arithmetic control portion 40 collates the identification information received from the transmitter-receiver portion 18 with the identification information read from the memory 43. In a case where a result of the collation reveals that the received identification information is not matched with the read identification information, the arithmetic control portion 40 inhibits the driving of the apparatus and also causes the display unit 45 to inform an operator of the result by indicating that the identification information is not matched. That is, in a case where the received information on the intended use of the blade unit 100 (the blade 20) is not matched with the read information on the intended use of the blade unit (the blade 20) corresponding to the selected technique, where the transmitter-receiver portion 18 does not receive the identification information or the like, the arithmetic control portion 40 inhibits the driving of the pump 41 and the motors 11 and 12.

On the other hand, in a case where the received identification information is matched with the read identification information, the arithmetic control portion 40 allows the driving of the apparatus, that is, drives the pump 41 and the motors 11 and 12. Further, the arithmetic control portion 40 causes the display unit 45 to inform an operator of this result by indicating that the identification information is matched. The arithmetic control portion 40 sets the oscillation frequency and the translation speed of the blade unit 100 (the blade 20) according to the drive program read from the memory 43. The driving of the motors 11 and 12 is controlled according to the set oscillation frequency and the set translation speed- In a case where detail setting of the oscillation frequency and the translation speed is necessary, such setting can be preliminarily performed by using various switches of the switch portion 44. Information on the driving conditions set in this manner is written to the IC chip of the communicating portion 25 by using the transmitter-receiver portion 18. The information on the driving conditions set in this manner is also written to the memory 43 together with the identification information of the blade unit 100 (the blade 20) .

In a case where the identification information is matched and where the allowance for driving the apparatus is obtained, the ring 31 is placed on the patient's eye. Then, the pump 41 is activated and sucks the air in the space S between the ring 31 and the patient's eye to thereby suction-fix the ring 31 to the patient' s eye. Subsequently, the foot switch 42 is operated to cause the arithmetic control portion 40 to rotate-drive the motors 11 and 12.

The blade unit 100 (the blade 20) is laterally oscillated by rotate-driving of the motor 12. Further, the blade unit 100 (the blade 20) is moved (translated) together with the head portion 2 as one unit in the direction of incision of the cornea (or the direction of separation of the epithelium) by rotate-driving of the motor 11, so that a flap is formed.

Incidentally, the information on the driving conditions, which is written to (or stored in) the IC chip, is read by being associated with the identification information. Consequently, the driving conditions, under which the blade unit 100 (the blade 20) are used, can be known.

Additionally, although the oscillation frequency and the translation speed of the blade unit 100 (the blade 20) are employed as the driving conditions in this embodiment, the driving conditions are not limited thereto. A suction pressure and a suction time may be employed as the driving conditions.

## Claims

1. A corneal surgical apparatus for forming a flap in a cornea, comprising:
a blade unit including a blade, and an identification information communicating portion that transmits identification information;
a head portion that holds the blade unit detachably;
an oscillation mechanism portion that oscillates the blade unit, which is held by the head portion, in a direction of a width of the blade;
a receiving portion that receives the identification information transmitted from the identification information communicating portion;
a storage portion that stores the identification information; and
a control portion that collates the identification information received by the receiving portion with the Identification information stored in the storage portion, and controls the driving of the oscillation mechanism portion based on a result of collation.

2. The corneal surgical apparatus according to claim 1, wherein the identification information communicating portion transmits intended-use information of the blade unit as the identification information, and the storage portion stores the intended-use information of the blade unit as the identification information.

3. The corneal surgical apparatus according to claim 2 further comprising a selection switch for selecting a corneal surgical technique,
wherein the control portion reads the intended-use information from the storage portion based on the selected technique and collates the received intended-use information with the read intended-use information.

4. The corneal surgical apparatus according to claim 1, wherein the identification information communicating portion transmits manufacturing information of the blade unit as identification information, and the storage portion stores the manufacturing information of the blade unit as identification information.

5. The corneal surgical apparatus according to claim 1 further comprising a notification portion,
wherein the control portion controls the notification portion to notify a result of collation.

6. The corneal surgical apparatus according to claim 1 further comprising a translation mechanism portion that moves the blade unit, which is held by the head portion, in a direction in which the flap is to be formed,
wherein the control portion controls driving of the translation mechanism portion based on a result of collation.

7. The corneal surgical apparatus according to claim 6, wherein
the identification information communicating portion transmits driving condition information including at least one of an oscillation frequency and a translation speed of the blade unit,
the receiving portion receives the driving condition information transmitted from the identification information communicating portion, and
the control portion controls driving of at least one of the oscillation mechanism portion and the translation mechanism portion based on driving condition information received by the receiving portion.

8. A blade unit attached to a corneal surgical apparatus for use in forming a flap in a cornea, comprising:
a blade;
a transmission member that is fixed to the blade and transmits to the blade impetus applied from the corneal surgical apparatus and causes the blade to oscillate in a direction of a width of the blade when the blade unit is attached to the corneal surgical apparatus; and
an identification information communicating portion that is provided in the transmission member and transmits identification information.

9. The blade unit according to claim 8, wherein the identification information communicating portion transmits at least one of intended-use information and manufacturing information of the blade unit.

10. The blade unit according to claim 8, wherein the identification information communicating portion transmits driving-condition information including an oscillation frequency of the blade unit.
